# EUROPÄISCHE PATENTSCHRIFT

(11) **EP 2 515 779 B1**
(45) Veröffentlichungstag und Bekanntmachung des Hinweises auf die Patenterteilung: **02.03.2016**
(21) Anmeldenummer: 10805682.1
(22) Anmeldetag: 21.12.2010
(51) Int. Cl.: A61B 17/80, A61B 17/86

(54) **KNOCHENPLATTENSYSTEM FÜR DIE OSTEOSYNTHESE**
BONE PLATE SYSTEM FOR OSTEOSYNTHESIS
SYSTÈME DE PLAQUES OSSEUSES POUR OSTÉOSYNTHÈSE

(30) Priorität: 22.12.2009 DE 102009060396; 20.04.2010 DE 202010005260 U
(43) Veröffentlichungstag der Anmeldung: 31.10.2012
(73) Patentinhaber: Merete Medical GmbH, 12247 Berlin (DE)
(72) Erfinder: CLASBRUMMEL, Bernhard, 72336 Balingen (DE); KRANZ, Curt, 14163 Berlin (DE)
(74) Vertreter: Bittner, Thomas L.
(86) Internationale Anmeldenummer: PCT/DE2010/075167
(87) Internationale Veröffentlichungsnummer: WO 2011/076205

(56) Entgegenhaltungen:
- EP-A2- 1 702 577
- WO-A1-2007/025520
- FR-A1- 2 886 535
- US-A1- 2004 215 192
- US-A1- 2007 123 885

## Beschreibung

Die Erfindung betrifft Knochenplattensystemen für die Osteosynthese.

### Hintergrund der Erfindung

Knochenplattensysteme dienen dem winkelstabilen Fixieren einer Knochenplatte mittels zugeordneter Schrauben zur Osteosynthese am menschlichen oder tierischen Körper. Zahlreiche Knochenplatterisysteme sind bekannt, die üblicherweise eine Knochenplatte mit einer Anordnung von mehreren Durchgangslöchern sowie zugehörige Schrauben umfassen. Hierbei können zusätzlich zu sogenannten Knochenschrauben, also Schrauben, die beim Fixieren in den Knochen eingedreht werden, Befestigungsschrauben vorgesehen sein, die ihrerseits nicht in den Knochen eingedreht werden, sondern vielmehr in ein in der Knochenplatte gebildetes Gewinde. Derartige Befestigungsschrauben dienen dann beispielsweise zur Fixierung der Knochenschrauben (vgl. zum Beispiel die Dokumente EP 1 702 577 A2, WO 2006/014436 A1 sowie AT 406 446 B). Auch bei dem System für eine Halswirbelsäule im Dokument DE 698 35 968 T2 wird eine Befestigungsschraube zum Fixieren mehrer Knochenschrauben genutzt.

Ein auf einem ähnlichen Konstruktionsprinzip beruhendes Knochenplattensystem ist in dem Dokument US 2004/0215192 A1 offenbart. Nebeneinander eingeschraubte Knochenschrauben werden mit Hilfe einer zusätzlichen Fixierschraube gesichert, welche ihrerseits in eine zugeordnete Gewindebohrung in der Knochenplatte eingeschraubt ist und im eingeschraubten Zustand die beiden Knochenschrauben stabilisiert.

Auch im Dokument FR 2 886 535 ist ein Knochenplattensystem mit einer ein Langloch enthaltenden Knochenplatte offenbart.

Winkelstabile Platten-Schrauben-Verbihdungen an Osteosyntheseplatten haben den Vorteil einer besseren Verankerung der Knochenplatte am den Knochen. Dies ist besonders bei gelenknahen Knochenbrüchen von Vorteil, da auf diese Art und Weise gelenknahe Knochenfragmente besser gefasst und fixiert werden können. Der Vorteil winkelstabiler Platten-Schrauben-Verbindungen gewinnt bei osteoporotischen gelenknahen Knochenbrüchen an Bedeutung, da Knochenschrauben ohne Winkelstabilität einen osteoporotischen Knochen schlechter fixieren können.

Winkelstabile Platten-Schrauben-Verbindungen können in monoaxiale und polyaxiale Platten-Schrauben-Verbindungen unterteilt werden.

Beispiele monoaxialer winkelstabiler Platten-Schrauben-Verbindurigen sind im Dokument DE 10 2005 043 285 B3 ausgeführt. Diese Systeme sind dadurch gekennzeichnet, dass sie Schraubenköpfe mit Außengewinde aufweisen, welche in korrespondierende. Innengewinde von Platten greifen. Wird eine Schraube während einer Operation eingeschraubt, bewirkt der Formschluss zwischen Außengewinde des Schraubenkopfes und Innengewinde der Knochenplatte eine winkelstabilen Platten-Schrauben-Verbindung während der letzten Umdrehungen des Einschraubvorgangs.

Weiterhin wurden Knochenplattensysteme vorgeschlagen, bei denen Knochenschrauben hinsichtlich ihrer Schwenk- oder Winkelstellung in Bezug auf die Knochenplatte beim Einsatz variabel sind. Ein solches Knochenplattensystem ist beispielsweise in dem Dokument DE 10 2006 000 948 A1 beschrieben. Im Dokument WO 2007 /025520 A1 ist eine Knochenplatte mit mindestens einer Schraube zur winkelstabilen Fixierung offenbart. Ein weiteres Beispiel einer polyaxialen winkelstabilen Platten-Schrauben-Verbindungen ist in DE 10 2005 042 766 B4 offenbart. Durch die dort beschrieben Ausbildung eines Innengewindes aus sechs Innengewindesäulen gelingt es, Kugelkopfschrauben mit einem besonderen Außengewinde in polyaxialer Richtung einzuschrauben und während der letzten Umdrehungen des Einschraubvorgangs winkelstabil zu fixieren. Aufgrund klinischer Vorteile einer polyaxialen winkelstabilen Fixierungsmöglichkeit haben sich derartige Platten-Schrauben-Systeme im klinischen Alltag immer mehr durchgesetzt.

Nachteilig an bekannten winkelstabilen Knochenplattensystemen ist die fehlende Möglichkeit, Knochenfragmente während des Vorgangs des Festdrehens an die Knochenplatte zu ziehen, da sich der Schraubenkopf aufgrund seines Gewindes am Schraubenkopf mit dem Gewinde in der Knochenplatte während der letzten Umdrehung verklemmt. Ein Heranziehen von Fragmenten in Gelenknähe ist jedoch häufig erforderlich, um eine bessere Wiederherstellung der ursprünglichen Anatomie zu erreichen. Des Weiteren fehlen für die Versorgung von geschwächten osteoporotischen Knochen Plattensysteme mit Möglichkeiten, mehrere Schrauben auf engem Raum winkelstabil an oder in einer Knochenplatte zu fixieren.

Im Dokument US 2007/0123885 A1 ist ein Knochenplattensystem beschrieben, bei dem die Knochenplatte endseitig über einen Durchbruch verbundene Durchgangslöcher aufweist, in die Knochenschrauben benachbart eingeschraubt werden, derart, dass die Schraubenköpfe in Kontakt kommen.

### Zusammenfassung der Erfindung

Aufgabe der Erfindung ist es, neue Technologien auf dem Gebiet von Knochenplattensystemen zur Osteosynthese anzugeben, die vom Anwender flexibel einsetzbar sind und über eine verbesserte Praktikabilität im Einsatz sowie optimierte Winkelstabilität für die Knochenschrauben verfügen.

Diese Aufgabe wird erfindungsgemäß durch ein Knochenplattensystem zur Osteosynthese nach dem unabhängigen Anspruch 1 gelöst. Vorteilhafte Ausgestaltungen der Erfindung sind Gegenstand von abhängigen Unteransprüchen.

Die Erfindung umfasst den Gedanken eines Knochenplattensystems zur Osteosynthese mit einer Knochenplatte, einer Schwenkschraube, einer Klemmschraube, einem Schwenkloch, welches in der Knochenplatte als Durchgangsloch zur polyaxialen Aufnahme der Schwenkschraube gebildet ist, und einem dem Schwenkloch zugeordneten Klemmloch, welches in der Knochenplatte als weiteres Durchgangsloch zur Aufnahme der Klemmschraube gebildet ist, wobei im eingeschraubten Zustand die Schwenkschraube und die Klemmschraube mehrdimensional winkelstabil fixiert sind, indem Schraubenköpfe der Schwenkschraube und der Klemmschraube untereinander sowie mit der Knochenplatte gegen eine Relativbewegung gesichert sind, und wobei die Schwenkschraube und die Klemmschraube jeweils als eine Knochenschraube ausgeführt sind.

Bei dem vorgeschlagenen Knochenplattensystem werden Knochenschrauben, also die hinsichtlich ihrer Schwenkstellung zur Knochenplatte einstellbare Schwenkschraube und die bezüglich ihrer Relativlage zur Knochenplatte im Wesentlichen festgelegte Klemmschraube, die auch als Fixier- oder Fixschraube bezeichnet werden kann, hinsichtlich ihrer räumlichen Winkelstellung zueinander und zur Knochenplatte im eingedrehten Zustand mehrfach stabilisiert, indem sowohl die Schraubenköpfe der beiden Schrauben miteinander als auch die Schraubenköpfe mit den Randbereichen der Durchgangslöcher in der Knochenplatte und somit mit der Knochenplatte selbst gegen eine Relativbewegung gesichert sind. Die Sicherung gegen Relativbewegung erfolgt vorzugsweise mittels Reib- und / oder Formschluss, zum Beispiel mithilfe Verklemmen. Der eingeschraubte Zustand bedeutet, dass sowohl Schwenkschraube als auch Klemmschraube in den Knochen eingeschraubt sind, da beide Schrauben als Knochenschraube ausgeführt sind, was insbesondere durch ein entsprechendes Knochengewinde auf dem jeweiligen Schraubenschaft zum Ausdruck kommt.

In einer Ausführungsform dient das weitere Durchgangsloch der monoaxialen Aufnahme der Klemmschraube.

Die Schwenkschraube und / oder die Klemmschraube können in einer Ausgestaltung einen Innenmehrkant oder eine sternartige Ausnehmung im jeweiligen Schraubenkopf aufweisen. Andere Schrauben-Mitnahmeprofile wie beispielsweise nach Phillips, Pozidriv, Torx, Vierkant, Tri-Wing, Torq-Set oder Spanner können angewendet werden.

Die Elemente des Knochenplattensystems, also insbesondere die Knochenplatte und / oder die Schwenkschraube und / oder die Klemmschraube, sind bevorzugt aus einer Titanlegierung hergestellt.

Die gegenseitige Abstützung oder Verklemmung der Schraubenköpfe untereinander sowie mit der Knochenplatte selbst erlaubt es, auf ein zusätzliches Fixiersystem der Knochenschrauben an der Knochenplatte zu verzichten, wie dieses bei bekannten Systemen im Stand der Technik vorgesehen ist.

Eine bevorzugte Weiterbildung der Erfindung sieht vor, dass das Schwenkloch mit einer sich zur Oberseite der Knochenplatte hin öffnenden Kugelkopfaufnahme gebildet und die Schwenkschraube einen zugeordneten Kugelkopf aufweist, der im eingeschraubten Zustand der Schwenkschraube wenigstens teilweise in der Kugelkopfaufnahme des Schwenkloches angeordnet ist.

Bei einer zweckmäßigen Ausgestaltung der Erfindung kann vorgesehen sein, dass die Kugelkopfaufnahme wenigstens abschnittsweise mit einer im Wesentlichen glatten Oberfläche und / oder wenigstens abschnittsweise mit einer Oberflächenkontur gebildet ist. Glatte Oberflächenabschnitte haben den Vorteil, dass die Schwenkstellungen der Schwenkschraube im Schwenkloch innerhalb der konstruktiv gegebenen Grenzen beliebig feinstufig gewählt werden können. Oberflächenkonturen im Bereich der Kugelkopfaufnahme können die Winkelstabilität in der genutzten Schwenkstellung der Schwenkschraube zusätzlich unterstützen. Zweckmäßig sieht eine Weiterbildung der Erfindung vor, dass das Klemmloch ein Innengewinde aufweist. Das Innengewinde ist in einem Teilabschnitt des Klemmloches oder das Klemmloch vollständig ausfüllend gebildet. Das Klemmloch ist auf diese Weise als ein Schraubloch ausgeführt. Das Innengewinde kann ein Innenspitzgewinde sein. Das Klemmloch kann ganz oder teilweise zylindrisch gebildet sein, so dass wenigstens ein zylindrischer Gewindeabschnitt hergestellt ist. Auch ein konischer Abschnitt kann im Klemmloch vorgesehen sein, vorzugsweise auf einer vom Knochen abgewandten Seite der Knochenplatte. Auf diese Weise ist ein konischer Gewindeabschnitt ausbildbar. Weist die Klemmschraube einen Schraubensenkkopf auf, ist dieser in einer Ausgestaltung ganz oder teilweise formschlüssig in dem konischen Abschnitt aufgenommen.

Eine bevorzugte Weiterbildung der Erfindung sieht vor, dass der Schraubenkopf der Klemmschraube ein Außengewinde aufweist. Das Außengewinde ist korrespondierend zum Innengewinde des Klemmloches gebildet, so dass das Außengewinde beim Eindrehen der Klemmschraube sich wenigstens teilweise in das Innengwinde eindreht.

Eine Fortbildung der Erfindung kann vorsehen, dass der Schraubenkopf der Schwenkschraube eine Gewindekontur aufweist, die mit im Wesentlichen horizontal umlaufenden Rillen, einem Rechts- sowie einem Linksgewinde gebildet ist, das Außengewinde des Schraubenkopfes der Klemmschraube im eingeschraubten Zustand der Klemmschraube wenigstens teilweise in das Innengewinde des Klemmloches eingedreht ist und im eingeschraubten Zustand Oberflächenabschnitte der Gewindekontur am Schraubkopf der Schwenkschraube und des Außengewindes am Schraubenkopf der Klemmschraube formschlüssig ineinander greifen. Die Winkelstabilität wird insbesondere dadurch unterstützt, dass Gewindeabschnitte der beiden Schraubenköpfe wenigstens im vollständig eingeschraubten Zustand formschlüssig ineinander greifen. Die mit umlaufenden Oberflächenrillen sowie Rechts- als auch Linksgewinde gebildete Gewindekontur am Kugelkopf der Schwenkschraube ermöglicht es, dass die Gewindekontur in beliebigen erlaubten Schwenk- oder Winkelstellungen der Schwenkschraube hinsichtlich der Knochenplatte mit einem Abschnitt des konischen Außengewindes am Schraubenkopf der Klemmschraube mindestens teilweise formschlüssig ineinandergreifen kann. Das Rechts- und / oder das Linksgewinde am Kugelkopf können in einer Ausgestaltung mehrgängig ausgeführt sein.

Bei einer Ausgestaltung der Erfindung kann vorgesehen sein, dass das Außengewinde am Schraubenkopf der Klemmschraube ein konisches Außengewinde ist, welches sich am Schraubenkopf in Einschraubrichtung verjüngt. Diese Ausführungsform unterstützt das klemmende Eindrehen in das Innengewinde, wobei die Klemmwirkung insbesondere bei einem zylindrischen Innengewinde optimiert ist.

Eine Weiterbildung der Erfindung kann vorsehen, dass bei der Klemmschraube das konische Außengewinde am Schraubenkopf mit einer Gewindesteigung gebildet ist, die geringer als die Gewindesteigung am Schaft der Klemmschraube ist. In einer Ausgestaltung sind die Gewindesteigungen so ausgeführt, dass das konische Außengewinde am Schraubenkopf ein Feingewinde und das Knochengewinde am Schaft der Klemmschraube ein Grobgewinde ist.

Eine vorteilhafte Ausführungsform der Erfindung sieht vor, dass das zwischen dem Schwenkloch und dem dem Schwenkloch zugeordneten Klemmloch ein Lochübergang mit einem Durchbruch gebildet ist. In einer Ausgestaltung kann auf diese Weise eine Art Langloch mit wahlweise schmalem Übergangsbereich gebildet werden.

Bevorzugt sieht eine Fortbildung der Erfindung vor, dass die Knochenplatte mit einem an das Klemmloch angrenzenden Dehnungsbereich gebildet ist, derart, dass die Knochenplatte beim Einschrauben der Klemmschraube in das Innengewinde des Klemmloches dem Einschraubdruck aufgrund der Wechselwirkung zwischen dem Außengewinde und dem Innengewinde zumindest teilweise nachgebend und frei von plastischer Verformung verformbar ist. Auf diese Weise können sich beim Eindrehen des Schraubenkopfes mit Außengewinde in das Innengewinde Bereiche des Klemmloches nicht plastisch, insbesondere elastisch, verformen, derart, dass sich das Klemmloch an die Form des Schraubenkopfes anpasst und die Schraube aufgrund der Umfangsspannung gegen das selbständige Herausdrehen behindert wird. Die den Dehnungsbereich vorsehende Ausgestaltung wird bevorzugt derart ausgeführt, dass das Außengewinde als konisches Außengewinde und das Innengewinde als zylindrisches Innengewinde gebildet sind.

Die Klemmschraube kann mit dem Schraubenkopf wahlweise vollständig in das Durchgangsloch eingedreht werden. Der Schraubenkopf wird hierbei durch die elastische Spannung in jeder Position einerseits drehsicher gehalten, andererseits ist aber die Knochenplatte auch nicht in ihrem plastischen Bereich irreversibel verformt, d. h. überdehnt. Auf diese Weise lässt sich erreichen, dass die Klemmschraube feinfühlig genau soweit eingedreht werden kann, wie es erforderlich ist, die Knochenplatte fest am Knochen anliegend zu fixieren und die Klemmung mit der Schwenkschraube zu erreichen, wobei gleichzeitig sichergestellt werden kann, dass der Schraubenkopf in seinem erhabensten Bereichen auch die Knochenplatte nicht überragt, sodass die fixierte Knochenplatte insgesamt - mit der Klemmschraube - eine im Wesentlichen sich an den Knochen ohne Erhebungen glatt anschmiegende Einheit bildet. Es kann in einer Ausführungsform vorgesehen sein, dass der angrenzende Dehnungsbereich mindestens teilweise als ein Steg oder Teil eines Ringes ausgebildet ist.

Bei einer zweckmäßigen Ausgestaltung der Erfindung kann vorgesehen sein, dass bei der Klemmschraube der Schraubenschaft eine Länge aufweist, die gleich der oder kürzer als die Länge des Schraubenschaftes der Schwenkschraube ist. In einer Ausgestaltung beträgt die Länge des Schaftes der Klemmschraube höchstens die Hälfte, bevorzugt höchstens ein Drittel der Länge des Schaftes der Schwenkschraube. Die gegenüber dem Schraubenschaft der Schwenkschraube verkürzte Länge des Schaftes der Klemmschraube unterstützt insbesondere die Vielgestaltigkeit der Schwenk- oder Winkelstellungen der Schwenkschraube bezüglich der Knochenplatte. So kann der Schaft der Schwenkschraube in einer Ausführungsform auch in einem Bereich unterhalb des unteren Schaftendes-der Klemmschraube geschwenkt werden.

Eine vorteilhafte Ausführungsform der Erfindung sieht vor, dass bei der Klemmschraube ein Knochengewinde auf dem Schraubenschaft benachbart zum Schraubenkopf mit einem sich erweiternden Abschnitt und sich an den Gewindegrund des konischen Außengewindes im Wesentlichen anschließenden Übergangsbereich gebildet ist.

Bevorzugt sieht eine Fortbildung der Erfindung vor, dass in der Knochenplatte mindestens ein weiteres dem Schwenkloch oder dem Klemmloch entsprechend ausgeführtes und zum Schwenkloch und zum Klemmloch korrespondierendes Durchgangsloch gebildet ist, dass in das weitere Durchgangsloch eine weitere Knochenschraube eingeführt ist, die der Schwenkschraube oder der Klemmschraube entsprechend ausgeführt ist, und dass der Schraubenkopf der weiteren Knochenschraube im eingeschraubten Zustand mit den Schraubenköpfen, beispielsweise dem Kugelkopf und dem Schraubenkopf mit konischem Außengewinde, sowie der Knochenplatte verklemmt ist. Bei dieser Ausführungsform ist eine Anordnung von wenigstens drei einander zugeordneten Durchgangslöchern gebildet, in die eine jeweilige Knochenschraube eingedreht ist. Es können beliebige Kombinationen von Schwenkschrauben und Klemmschrauben vorgesehen sein, wobei wahlweise Gewindeabschnitte der Schraubenköpfe der beteiligten Knochenschrauben paarweise formschlüssig ineinandergreifen und sich so aufeinander abstützen und gemeinsam an der Knochenplatte gegen eine Relativbewegung gesichert sind.

Nachfolgend werden bevorzugte Ausführungsformen der Erfindung näher erläutert.

Das Schwenkloch und das zugeordnete Klemmloch können Teil einer sogenannten Plattenlochgruppe mit weiteren Durchgangslöchern sein oder diese bilden. Die Knochenplatte kann mehrere Plattenlochgruppen aufweisen. Zusätzliche Durchgangslöcher können als Rund- oder Langlöcher zur passgenauen Aufnahme von Rund-, Senk-, Kugel-, Linsen-, Birnen- oder Konuskopfschrauben geformt sein. Eine für die Knochenplatte besonders vorteilhafte Schwenkschraube weist einen kugelförmigen Schraubenkopf auf, der am Kopfende (Nordpol) abgeflacht ist.

Eine bevorzugte Klemmschraube ist als Senkkopfschraube mit einem zylindrischen Gewinde unterhalb des Senkkopfes ausgebildet. Die Form der Unterfläche der Senkkopfschraube kann hierbei zur Verbesserung der Kontaktfläche gerundet und passend zur Kugelform des Schwenkschraubenkopfes ausgebildet sein. Die Länge des zylindrischen Gewindes des Schraubenbolzens beträgt ein Vielfaches der Plattendicke, zum Beispiel das 0,9-fache. Hierdurch kann das zylindrische Gewinde der Klemmschraube zunächst sicher im zylindrischen Gegengewinde der Platte fassen und im weiteren Verlauf des Eindrehens die Schwenkschraube an ihren geplanten Platz drücken, so dass eine möglichst größe Klemmwirkung bereitgestellt ist. Dies ist besonders bei leicht verkanteten Schwenkschrauben von Vorteil. Bei dem Vorgang des Verklemmens überragt das Zylindergewinde der Klemmschraube geringfügig die Knochenplattenunterfläche, weswegen der Beginn des schraubenspitzenseitigen Zylindergewindes als selbstschneidendes Gewinde ausgebildet sein kann, so dass das Zylindergewinde geringfügig in den zu verschraubenden Knochen eindringen kann. Der weitere in der Regel längere schräubenspitzenseitige Anteil des Bolzens der Klemmschraube ist typischerweise wie eine Knochenschraube mit einem selbstschneidenden Gewinde ausgeführt.

Das Außengewinde der Klemmschraube, welches im Gegengewinde der Knochenplatte fassen soll, kann als Konusgewinde ausgebildet sein, wobei der Neigungswinkel des Konus zur Längsachse der Schraube geringer ausfällt als der Neigungswinkel des Senkkopfes.

Die Schwenkschraube kann bei der Anwendung zuerst polyaxial eingedreht werden, was es ermöglicht, Knochenfragmente zu fixieren und an die Platte zu ziehen. Anschließend kann die Klemmschraube eingedreht werden und gleichzeitig sich selbst und die Schwenkschraube während der letzten Umdrehungen winkelstabil fixieren. Hierbei wirkt die Klemmschraube selbst als monoaxiale winkelstabile Schraube.

Die Polyaxialität der Schwenkschraube wird in einer Ausgestaltung durch den äußeren Rand des Schraubenbolzens der Schwenkschraube begrenzt, welcher in geschwenktem Zustand an den unteren Rand (entspricht der dem Schraubenkopf abgewandten Knochenplattenseite) des Schwenkloches der Platte stößt. Um eine hohe variable Einstellung der Schwenkschrauben-Längsachse zu erreichen, ist vorzugsweise der Bolzen in dem Bereich unterhalb des Schraubenkopfes von einem Gewinde befreit. Die Unterseite des Schwenkloches weist vorteilhaft ergänzend eine Fase auf, um einen Schwenkradius der Schwenkschraube zu erweitern. Hierdurch kann die Schraubenlängsachse variabel zur Lochachse bis zu einem möglichst hohen azimutalen Winkel eingebracht werden.

Um einer Zerstörung des konischen Gewindes der Klemmschraube durch Reibung an dem Kugelkopf der Schwenkschraube vorzubeugen, kann das Gewinde vorteilhaft abgerundete Spitzen aufweisen.

In einem weiteren Ausführungsbeispiel ist die Oberfläche der Schwenkschraube derart ausgebildet ist, dass die Rundkopffläche der Schwenkschraube senkrecht zum Äquator des Schraubenkopfes Längssegmente aufweist, beispielsweise zwölf Längensegmente. Jedes Längssegment ist mit einer Innenspitzgewindesäule ausgestaltet, um als formschlüssiges Widerlager für die Klemmschraube zu dienen. Somit kann eine winkelstabile formschlüssige Fixierung beider Schrauben wie folgt stattfinden. Die Klemmschraube weist ein konisches Außenspitzgewinde auf, welches einerseits eine Verklemmung mit der Platte über ein konisches Innenspitzgewinde des Klemmloches formschlüssig erlaubt. Die Schwenkschraube ist über deren Innengewindesäulen am Schraubenkopf formschlüssig fixiert. Hierbei sind die Innengewindesäulen an der Schwenkschraube zum Beispiel derart ausgebildet, dass die Oberfläche des Schraubenkopfteils der Schwenkschraube noch genügend Kugelfläche aufweist, so dass beim Festdrehen der Schwenkschraube keine Gewindezerstörung oder Gratbildung auftritt.

In einer weiteren vorteilhaften Ausgestaltung kann die Fläche des Schwenkloches mit mindestens einer Innenspitzgewindesäule ausgestattet sein, um der Montage eine höhere Winkelstabilität zu verleihen. Da die Schwenkschraube ebenfalls mit vorzugsweise zwölf Innengewindesäulen ausgestattet sein kann, können die genannten Innenspitzgewindesäulen auf der Fläche des Schwenkloches vorteilhaft mit einer angepassten oder passfähigen Gewindeform ausgebildet werden.

Mithilfe derartiger auf Flächen von Schwenklöchern ausgebildeter Innengewindesäulen wird eine erhöhte Winkelstabilität erzielt. Dies kann bei Knochenplattensystemen zur Versorgung von beispielsweise Oberschenkelbrüchen vorteilhaft sein. Ein offensichtlicher Nachteil der Innengewindesäulen in der Fläche des Schwenkloches ist eine eingeschränkte Möglichkeit, Knochenfragmente mit der Schwenkschraube heranzuziehen, was Folge des Formschlusses der Schwenkschraube mit den genannten Innengewindesäulen ist. Dieser Nachteil, also eine eingeschränkte Möglichkeit, Fragmente heranzuziehen, kann in einer Ausgestaltung klinisch gleichwohl als Vorteil genutzt werden, indem schon nach dem Einbringen einer Schwenkschraube eine bestimmte Winkelstabilität erreicht wird. Nach Einbringen einer Klemmschraube wird eine höhere und robustere Winkelstabilität der Schwenkschraube mit Hilfe der Klemmschraube erreicht.

Mittels Variation der Fläche des Schwenkloches, sei es ohne oder mit mindestens einer Innenspitzgewindesäule, kann außerdem der Grad der Winkelstabilität der Schwenkschraube vorbestimmt werden, was eine vorteilhafte Anpassung an klinische Bedürfnisse gestattet.

Mit dem vorgeschlagenen Knochenplattensystem werden in seinen verschiedenen Ausgestaltungen gegenüber herkömmlichen Implantaten unterschiedliche Vorteile erreicht. Dies sind zum Beispiel ein oder mehrere der folgenden Vorteile:
- Verbesserter Halt eines divergierenden winkelstabilen Knochenschraubenpaares (oder mehrerer winkelstabiler Schrauben) gegenüber einer winkelstabilen Schraube.
- Durch verbesserten Halt der Knochenschrauben können kürzere oder schmalere Knochenplatten eingesetzt werden.
- Durch verbesserten Halt der Knochenschrauben ist das neue System besonders geeignet für die Osteosynthese von osteoporotischen Knochen.
- An Plattenenden können mehr winkelstabile Schrauben auf kleinem Raum untergebracht werden.
- Die Klemmschraube kann in kurzer Ausführung nur verklemmend wirken oder in längerer Ausführung als monoaxiale winkelstabile Schraube zusätzlich eingesetzt werden.
- Verbesserte Einsatzmöglichkeit im Falle von periprothetischen Frakturen.
- Die Schwenkschraube kann Knochenfragmente heranziehen und anschließend durch die Klemmschraube winkelstabil fixiert werden.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1a: einen Längsschnitt durch ein Knochenplattensystem mit einer Knochenplatte, einer Schwenkschraube und einer Klemmschraube,
- Fig. 1b: einen Längsschnitt durch die Schwenkschraube aus Fig. 1a,
- Fig. 1c: einen Längsschnitt durch die Klemmschraube aus Fig. 1a,
- Fig. 2a: einen Längsschnitt durch ein Knochenplattensystem mit einer Knochenplatte, einer Schwenkschraube und einer Klemmschraube nach einer weiteren Ausführung,
- Fig. 2b: einen Längsschnitt durch die Schwenkschraube von Fig. 2a,
- Fig. 2c: einen Längsschnitt durch die Klemmschraube von Fig. 2a,
- Fig. 3a: eine Draufsicht auf eine Knochenplatte mit einer Plattenlochgruppe,
- Fig. 3b: eine Draufsicht auf eine Knochenplatte mit einer Plattenlochgruppe nach einer weiteren Ausführung,
- Fig. 3c: eine Abwandlung der Knochenplatte von Fig. 3b,
- Fig. 4a: einen Längsschnitt durch eine Schwenkschraube mit abgeflachtem Kugelkopf und mit Innengewindesäulen auf der Kugelkopfoberfläche,
- Fig. 4b: eine Draufsicht der Schwenkschraube von Fig. 4a,
- Fig. 5a: einen Längsschnitt durch eine Klemmschraube mit konischem Senkkopf und Außenspitzgewinde,
- Fig. 5b: einen Längsschnitt durch eine Klemmschraube mit zylindrischem Außengewinde und Senkkopf mit kegelförmiger Unterfläche des Senkkopfes,
- Fig. 5c: einen Längsschnitt durch eine Klemmschraube mit zylindrischem Außengewinde und Senkkopf mit radiären Stegen an der Unterfläche des Senkkopfes,
- Fig. 6a: ein Knochenplattensystem mit einer geeigneten Schwenkschraube und der Klemmschraube aus Fig. 5c,
- Fig. 6b: einen Querschnitt durch das Klemmloch von Fig. 6a entlang der Linie A - B von Fig. 6a zur Aufnahme einer Klemmschraube von Fig. 5 b und 5 c,
- Fig. 7a: eine Draufsicht auf eine Knochenplatte mit einer Plattenlochgruppe,
- Fig. 7b: eine Abwandlung der Ausführung von Fig. 7a mit einer abgewandelten Plattenlochgruppe,
- Fig. 8: eine sehematisehe Darstellung eines weiteren Knochenplattensystems mit einer Knochenplatte und hieran winkelstabil fixierten Knochenschrauben nach einer weiteren Ausführungsform,
- Fig. 9: eine schematische Darstellung des Knochenplattensystems nach Fig. 8 mit verschiedenen Schwenkstellungen für eine als Schwenkschraube ausgeführte Knochenschraube,
- Fig. 10: eine schematische Darstellung eines Abschnitts eines anderen Knochenplattensystems, wobei eine Klemmschraube mit konischem Außengewinde am Schraubenkopf in ein zylindrisches Innengewinde teilweise eingedreht ist,
- Fig. 11: eine schematische Darstellung einer als Schwenkschraube ausgeführten Knochenschraube mit einer Gewindekontur am kugelförmigen Schraubenkopf und
- Fig. 12: eine schematische Darstellung einer Knochenschraube mit konischem Außengewinde am Schraubenkopf.

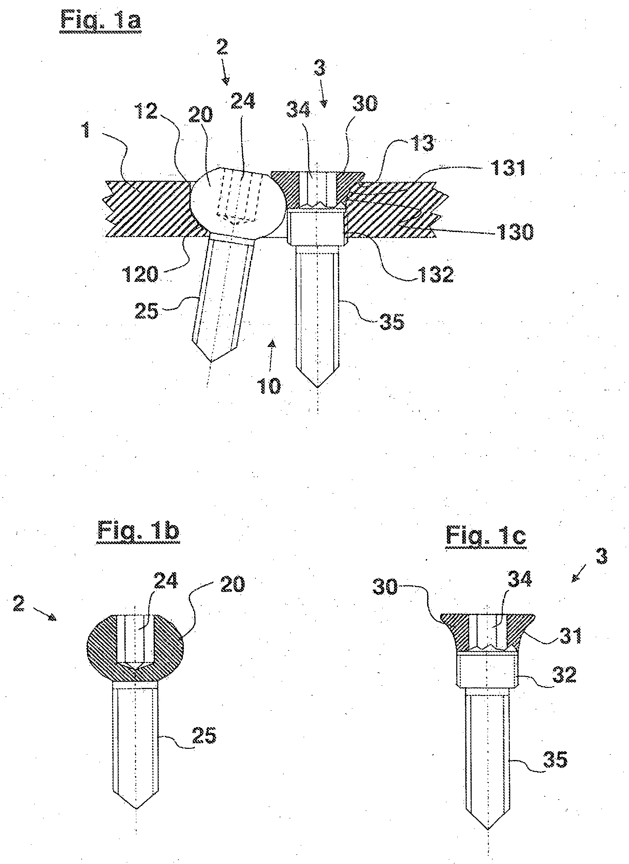

Fig. 1a zeigt einen schematischen Teilschnitt durch eine Knochenplatte 1 mit einer Plattenlochgruppe 10 mit einem Schwenkloch 12 und einem Klemmloch 13, die in einem vorbestimmten Abstand zueinander angeordnet sind, wobei in dem Schwenkloch 12 eine Schwenkschraube 2 und in dem Klemmloch 13 eine Klemmschraube 3 angeordnet sind. Das Schwenkloch 12 umfasst in einem einem Knochen zugewandten Bereich der Knochenplatte 1 eine Verjüngung 120, die wahlweise formschlüssig mit dem Schraubenkopf 20 der Schwenkschraube 2 ausgebildet ist, und das Klemmloch 13 umfasst eine Verjüngung 130, die wahlweise formschlüssig mit dem Schraubenkopf 30 der Klemmschraube 3 ausgebildet ist.

Bei der Ausführung von Fig. 1a umfasst die Schwenkschraube 2 einen kugelförmig ausgebildeten Schraubenkopf 20, der abgeflacht ist und außerdem einen Innensechskant 24 zum Drehen der Schraube mit einem geeigneten Werkzeug aufweist. Die in Fig. 1b ohne die Knochenplatte 1 dargestellte Schwenkschraube 2 weist ein Gewinde für eine Knochenschraube. 25 auf

Bei der Ausführung von Fig. 1a ist in dem Bereich der einem Knochen abgewandten Seite der Knochenplatte 1 des Klemmlochs 13 ein bogenförmiges Senkloch 131 ausgebildet, welches mit einem passend geformten bogenförmigen Senkkopf 31 des Schraubenkopfes 30 der Klemmschraube 3 korrespondiert, wobei an die Verjüngung 130 des Klemmloches 13 anschließend.das Klemmloch 13 zylindrisch ausgebildet ist und in dem zylindrisch ausgebildeten Bereich des Klemmlochs 13 ein zylindrisches Innenspitzgewinde 132 ausgebildet ist, das mit einem zylindrischen Außenspitzgewinde 32 der Klemmschraube 3 korrespondiert, das sich an dem Schraubenkopf 30 der Klemmschraube 3 nach unten anschließt.

Die Klemmschraube 3 ohne die Knochenplatte 1 mit dem Schraubenkopf 30, dem bogenförmigen Senkkopf 31 des Schraubenkopfes 30 und dem zylindrischen Außenspitzgewinde 32 ist schematisch in Fig. 1c dargestellt, wobei die Klemmschraube 3 außerdem in ihrem Schraubenkopf 30 einen Innensechskant 34 aufweist und ein Gewinde für eine Knochenschraube 35 umfasst.

Bei der Ausführung von Fig. 1 ist der Schraubenkopf 30 der Klemmschraube 3 formschlüssig zu dem Schraubenkopf 20 der Schwenkschraube 2 ausgebildet und dementsprechend auch das Klemmloch 13 mit der entsprechend ausgebildeten Verjüngung 130 formschlüssig mit dem Schraubenkopf 30 der Klemmschraube 3 ausgebildet.

Die Ausführung von Fig. 1 der Knochenplatte 1, der Plattenlochgruppe 10 und dem Schwenkloch 12 und dem Klemmloch 13 und der Schwenkschraube 2 und der Klemmschraube 3 ist besonders vorteilhaft, nachdem durch die formschlüssige Ausbildung des Schraubenkopfes 20 der Schwenkschraube 2 mit dem Schwenkloch 12 und dem Schraubenkopf 30 der Klemmschraube 3 und außerdem durch die formschlüssige Ausbildung des Schraubenkopfes 30 der Klemmschraube 3 mit dem Klemmloch 13 eine besonders stabile und dauerhafte Verklemmung von Knochenplatte 1, Schwenkschraube 2 und Klemmschraube 3 ermöglicht wird.

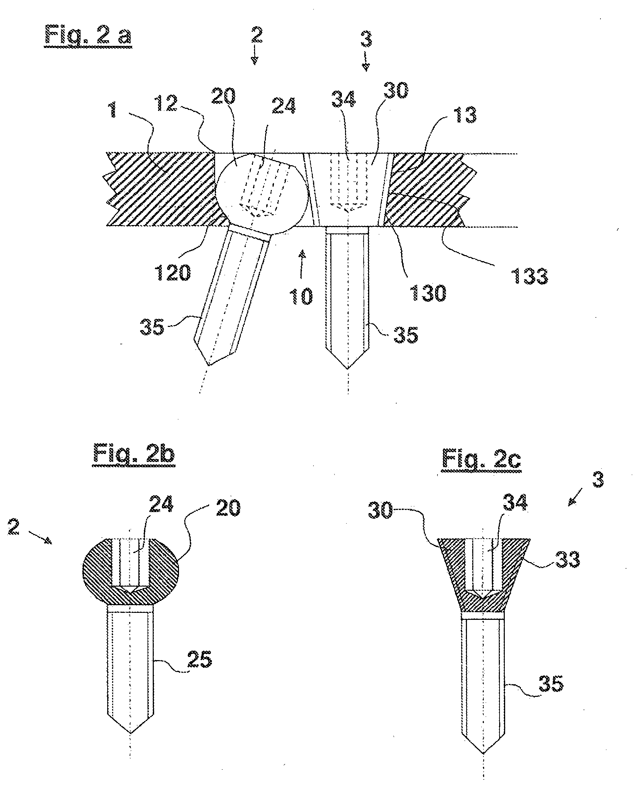

Fig. 2a zeigt eine Abwandlung der Knochenplatte 1 aus Fig. 1a mit der Schwenkschraube 2 und Klemmschraube 3, und Fig. 2b und 2c zeigen jeweils die Schwenkschraube 2 und die Klemmschraube 3 aus Fig. 2a.

Die Ausführung der Knochenplatte 1 in Fig. 2ä ist ähnlich ausgebildet wie die Knochenplatte 1 von Fig. 1a, wobei für identische und ähnliche Ausbildungen gleiche Bezugszeichen verwendet sind, und wobei für identische Ausbildungen auf die vorstehende Beschreibung von Fig. 1 verwiesen wird. Das Gleiche gilt für Ausbildungen der Schwenkschraube 2 und der Klemmschraube 3.

Im Unterschied zu der Ausbildung der Knochenplatte 1 von Fig. 1 ist bei der Ausführung von Fig. 2 die Verjüngung 130 des Klemmloches 3 durchgehend konisch ausgebildet und in dem konisch ausgebildeten Bereich des Klemmloches 13 ein Innenspitzgewinde 133 ausgebildet, das mit einem Außenspitzgewinde 33 der Klemmschraube 3 korrespondiert, das auf einem konisch ausgebildeten Senkkopf 30 der Klemmschraube 3 ausgebildet ist, der mit dem konisch ausgebildeten Klemmloch 13 korrespondiert.

In der Ausführung von Fig. 2 hat die Knochenplatte 1 gegenüber den Schraubenköpfen 20 und 30 der Schwenkschraube 2 und der Klemmschraube 3 außerdem eine Mächtigkeit, so dass die Schraubenköpfe 20 und 30 der Schrauben 2 und 3 jeweils vollständig in ihren jeweiligen Löchern 12 und 13 der Plattenlochgruppe 10 versenkbar sind, wodurch vorteilhaft eine ebene Oberseite der Knochenplatte 1 ohne Erhöhungen durch die Schraubenköpfe 20 und 30 bereitgestellt ist.

Die Ausführungen in den Fig. 1 und 2 haben gemeinsam, dass ein Abstand zwischen den Löchern 12 und 13 und die Abmessung der Plattenlochgruppe 10 und die Abmessungen der Schraubenköpfe 20 und 30 derart vorbestimmt gewählt sind, dass bei vollständig in die Knochenplatte 1 geschraubten Schrauben 2 und 3 eine winkelstabile Klemmwirkung der Schraubenköpfe 20 und 30 untereinander und der Schraubenköpfe 20 und 30 mit der Knochenplatte 1 bereitgestellt ist. Auch eine Kombination der Ausführungen von Fig. 1 und 2 kann vorgesehen sein, beispielsweise eine entsprechend dicker ausgebildete Knochenplatte 1 von Fig. 1a, so dass die Schraubenköpfe 20 und 30 der Ausführung von Fig. 1, vollständig in die Plattenlochgruppe 10 versenkbar sind.

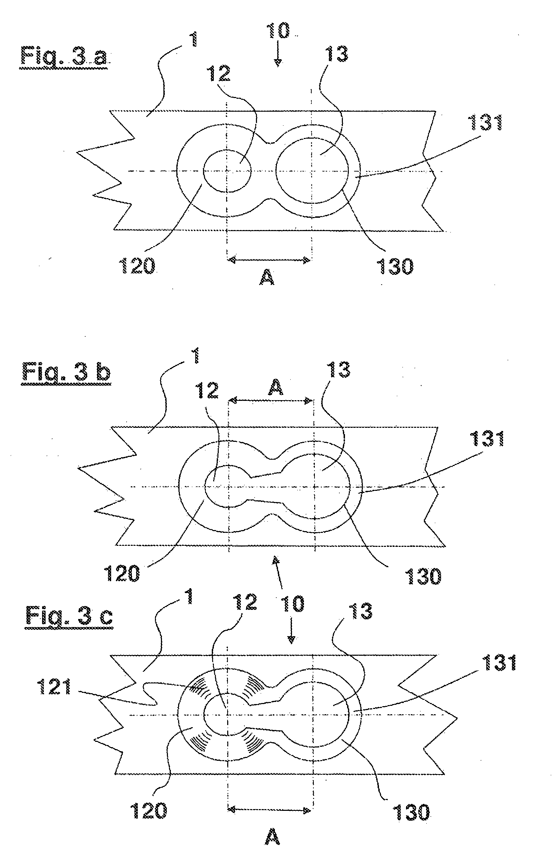

Fig. 3a, b und c zeigen jeweils eine schematische Draufsicht auf einen Ausschnitt einer Knochenplatte 1 mit einer Plattenlochgruppe 10 mit einem Schwenkloch 12 für eine Schwenkschraube 2 und einem Klemmloch 13 für eine Klemmschraube 3, wobei das Schwenkloch 12 und das Klemmloch 13 wenigstens teilweise ineinandergreifend ausgebildet sind, wobei außerdem das Schwenkloch 12 und das Klemmloch 13 in einem vorbestimmten Abstand A voneinander angeordnet sind, so dass bei vollständig in die Löcher 12 und 13 gedrehten Schrauben 2 und 3 die Schrauben 2 und 3 mit ihren Schraubenköpfen 20 und 30 untereinander und mit den Rändern der Löcher 12 und 13 der Knochenplatte 1 verklemmen und eine winkelstabile Fixierung von Schwenkschraube 2 und Klemmschraube 3 bereitgestellt ist.

Wie vorstehend anhand von Fig. 1 und 2 beschrieben umfassen die Löcher 12 und 13 hierbei in dem einem Knochen zugewandten Bereich der Knochenplatte 1 Verjüngungen 120 und 130, die geeignet korrespondierend mit den jeweiligen Schraubenköpfen 20 und 30 ausgebildet sind, und wobei an dem Klemmloch 13 ein bogenförmiges Senkloch 131 und ein zylindrisches Innenspitzgewinde 130 oder ein konisches Innenspitzgewinde 133 ausgebildet sein kann.

Erfindungsgemäß greifen die Löcher 12 und 13 zumindest in einem ersten Bereich der Knochenplatte 1 die einem Knochen abgewandt ist ineinander, wobei die Löcher 12 und 13 daran anschließend in dem einen Knochen zugewandten Bereich voneinander beabstandet sein können, was schematisch in der Ausführung von Fig. 3a dargestellt ist.

Fig. 3b zeigt eine Abwandlung der Knochenplatte 1 aus Fig. 3a, bei der die Plattenlochgruppe 10 vorteilhaft derart ausgebildet ist, dass die ineinandergreifenden Schraubenlöcher 12 und 13 ein zusammenhängendes durchgehendes Loch bilden. Fig. 3c zeigt eine besonders vorteilhafte Abwandlung der Ausführung von Fig. 3b, bei der in dem Schwenkloch 12 Innenspitzgewindesäulen 121 ausgebildet sind, die einen Form- und / oder Kraftschluss und ein Verklemmen des Schraubenkopfes 20 der Schwenkschraube 2 mit der Knochenplatte 1 unterstützen.

Für eine weitere Begünstigung der Haftreibung und des Verklemmens des Schraubenkopfes 20 der Schwenkschraube 2 mit der Knochenplatte 1 können außerdem Innenspitzgewindesäulen 21 auf der Oberfläche des Schraubenkopfes 20 der Schwenkkopfschraube 2 ausgebildet sein, die auch vorteilhaft korrespondierend zu den Innenspitzgewindesäulen 121 der Ausführung der Knochenplatte 1 von Fig. 3c ausgebildet sind.

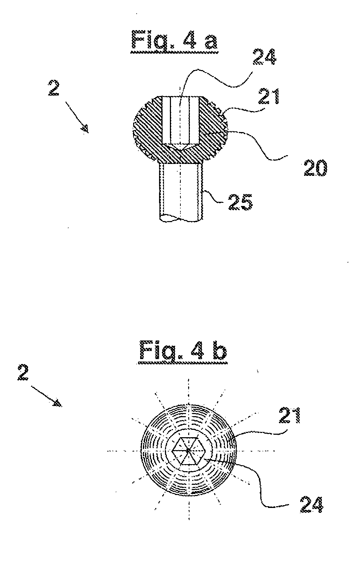

Eine schematische Darstellung eines mit Innenspitzgewindesäulen 21 versehenden Schraubenkopfes 20 einer Schwenkschraube 2 zeigt Fig. 4a. Fig. 4b zeigt den Schraubenkopf 20 aus Fig. 4a von oben. Natürlich begünstigt der mit Innenspitzgewindesäulen versehende Schraubenkopf 20 der Ausführung der Schwenkschraube 2 von Fig. 4 auch eine Haftreibung und Klemmwirkung mit der Knochenplatte 1 von Fig. 1, Fig. 2, Fig. 3a und 3b und mit der Klemmschraube 3 von Fig. 1 und Fig. 2.

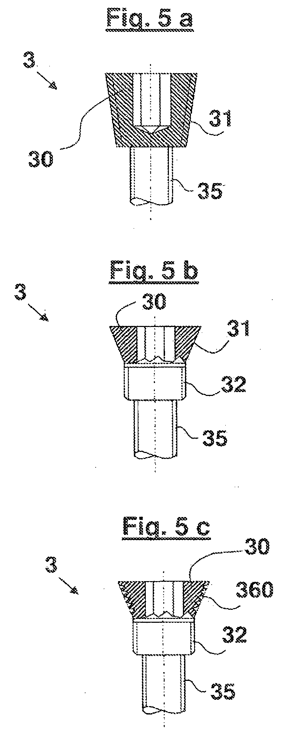

Fig. 5a zeigt eine schematische Teildarstellung einer für eine Knochenplatte 1 besonders geeignete Klemmschraube 3 mit dem Schraubenkopf 30 und einem an dem Schraubenkopf 30 ausgebildeten Außenspitzgewinde 33, wobei der Schraubenkopf 30 vollständig konisch ausgebildet ist und besonders für eine Knochenplatte 1 von Fig. 2a geeignet ist. Fig. 5b zeigt eine schematische Teildarstellung einer Schraube 3 mit dem Schraubenkopf 30, der in einem ersten Bereich konisch ausgebildet ist, an den sich ein zylindrisch ausgebildeter Bereich mit einem zylindrischen Außenspitzgewinde 32 anschließt, wobei die Klemmschraube 3 aus Fig. 5b besonders geeignet ist für eine in den Zeichnungen nicht dargestellte Kombination einer Knochenplatte 1 nach Fig. 1 a und Fig. 2a mit einem Klemmloch 13, das in einem ersten Bereich konisch ausgebildet ist und einem daran anschließenden Bereich zylindrisch ausgebildet ist, wobei der zylindrische Bereich des Klemmloches 13 mit einem entsprechenden Innengewinde 32 versehen ist.

Fig. 5c zeigt eine schematische Darstellung einer Abwandlung der Klemmschraube 3 aus Fig. 5b, bei der der Schraubenkopf der Klemmschraube 3 in einem ersten Bereich konisch ausgebildet ist und in einem zweiten anschließenden Bereich zylindrisch ausgebildet ist, wobei in dem zylindrisch ausgebildeten Bereich,ein zylindrisches Außenspitzgewinde 32 gebildet ist. Im Unterschied zu der Klemmschraube 3 von Fig. 5b umfasst die Klemmschraube 3 von Fig. 5c an ihrem konisch ausgebildeten Kopf 30 anstelle der konischen Oberfläche des Senkkopfes 31 der Klemmschraube 3 von Fig. 5b radiäre Außenstege 360, die eine besonders gute Haftreibung und Klemmwirkung zwischen dem Schraubenkopf 30 der Klemmschraube 3 mit der Knochenplatte 1 und dem Schraubenkopf 20 der Schwenkschraube 2 bereitstellen. Anstelle der radiären Außenstege 360 kann der Senkkopf mit einem konischen Außengewinde versehen sein.

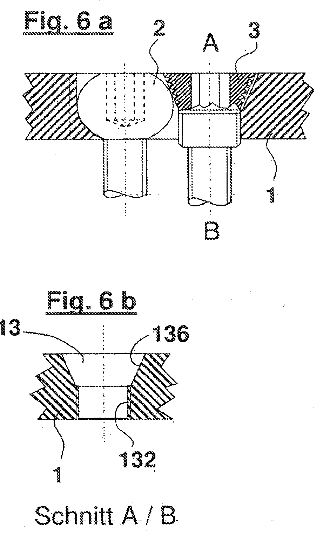

Fig. 6a zeigt eine schematische Teildarstellung einer Knochenplatte 1 nach einer weiteren Ausführung, die besonders für Fixschrauben 3 nach Fig. 5c geeignet ist, wobei bei der Knochenplatte 1 aus Fig. 6a das Klemmloch 13 in einem ersten Bereich konisch ausgebildet ist und in seinem konisch ausgebildeten Bereich ohne Gewinde 136 oder mit einem Innenspitzgewinde versehen sein kann. In einem an den ersten Bereich anschließenden zweiten Bereich ist das Klemmloch 13 zylindrisch ausgebildet und mit einem zylindrischen Innenspitzgewinde 132 versehen, das mit dem zylindrischen Außenspitzgewinde 32 der Klemmschraube 3 von Fig. 5c korrespondiert, wobei vorteilhaft die Höhe des zylindrischen Bereiches des Klemmlochs 13 der Knochenplatte geringer ist als die Höhe des zylindrischen Bereiches der Klemmschraube 3, so dass der zylindrische Bereich der Klemmschraube 3 bei vollständig in die Knochenplatte 1 gedrehter Klemmschraube 3 die einem Knochen zugewandte Seite der Knochenplatte 1 um einen vorbestimmten Betrag überragt. Fig. 6b zeigt hierzu einen Schnitt durch die Knochenplatte 1 entlang der Linie A - B von Fig. 6a.

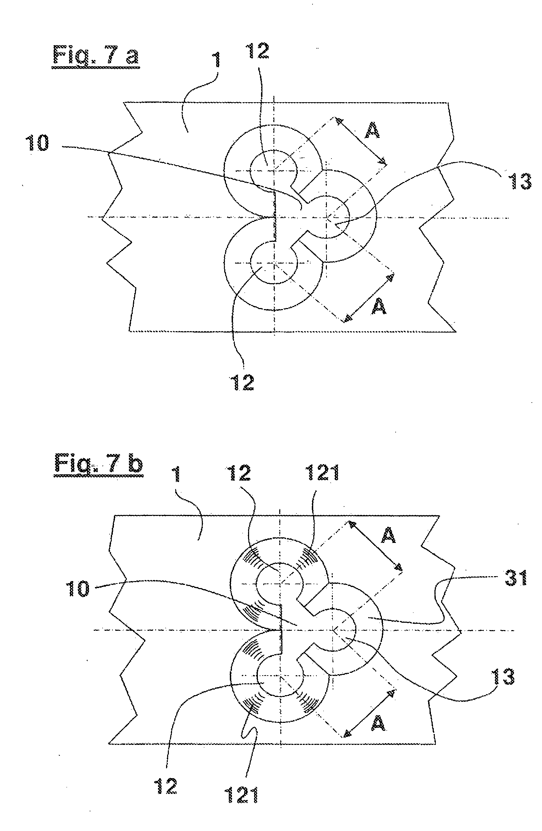

Fig. 7a zeigt eine schematische Draufsicht auf eine Knochenplatte 1 nach einer weiteren Ausführung mit einer Plattenlochgruppe 10, die zwei Schwenklöeher 12 für Schwenkschrauben umfasst und außerdem ein Klemmloch 13 für eine Klemmschraube 3 umfasst. Die Löcher 12 und 13 sind jeweils ineinandergreifend ausgebildet und in einem vorbestimmten Abstand A angeordnet. Bei der Ausführung von Fig. 7a ist die Plattenlochgruppe 10 beispielhaft und vorteilhaft derart ausgebildet, dass die ineinandergreifenden Schraubenlöcher 12 und 13 ein zusammenhängendes durchgehendes Loch bilden.

Wie bei der vorstehend beschriebenen Ausführung nach den Fig. 1, 2 und 6 ist bei der Ausführung von Fig. 7a der Abstand A zwischen Klemmloch 13 und Schwenkloch 12 derart gewählt, dass bei vollständig in die Löcher 12 und 13 gedrehten Schrauben 2 und 3 die Schraubenköpfe 20 und 30 der Schrauben 2 und 3 untereinander und mit der Knochenplatte 1 verklemmen, so dass eine winkelstabile Fixierung von Schwenkschraube 2 und Klemmschraube 3 bereitgestellt ist. Bei der Ausführung von Fig. 7a kann das Klemmloch 13 gemäß der Ausführung aus Fig. 1 oder Fig. 2 oder Fig. 6 ausgebildet sein. Das Schwenkloch 12 kann gemäß dem Schwenkloch 12 der Ausführung von Fig. 3b oder 3c ausgebildet sein, wobei das Schwenkloch Innenspitzgewindesäulen 121 umfassen kann, was schematisch in der Ausführung von Fig: 7b dargestellt ist. Eine Plattenlochgruppe 10 einer Knochenplatte 1 kann eine Vielzahl von Schwenklöchern 12 und / oder Klemmlöchern 13 umfassen, die jeweils ineinandergreifend ausgebildet sind.

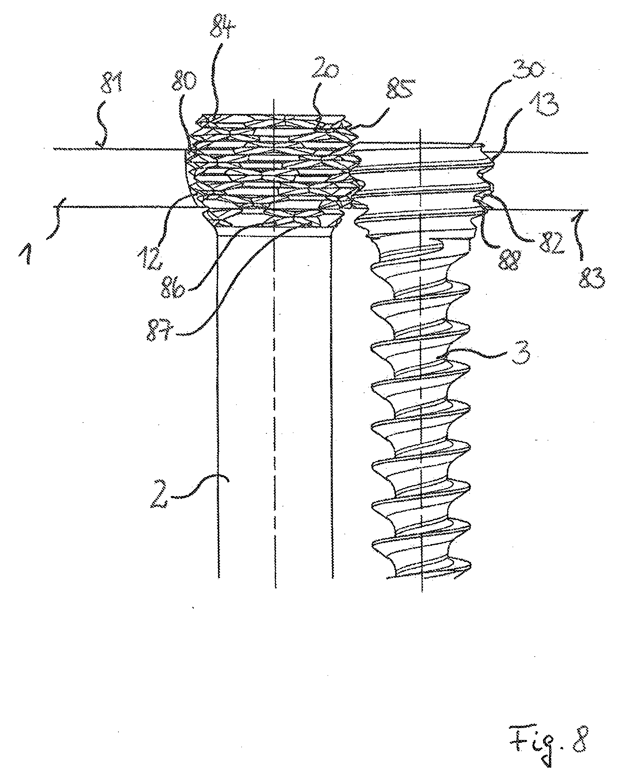

Fig. 8 zeigt eine schematische Darstellung eines Knochenplattensystems mit einer Knochenplatte 1 nach einer weiteren Ausführungsform, in welcher das Schwenkloch 12 sowie das dem Schwenkloch 12 zugeordnete Schraub- oder Klemmloch 13 gebildet sind. Das Schwenkloch 12 ist mit einer Kugelkopfaufnahme 80 hergestellt, die sich zur Oberseite 81 der Knochenplatte 1 öffnet. Die Kugelkopfaufnahme 80 ist in der dargestellten Ausführungsform mit einer im Wesentlichen glatten Innenoberfläche hergestellt.

Das Schraub- oder Klemmloch 13 weist, ähnlich zu dem zylindrischen Innenspitzgewinde 132, ein zylindrisches Innengewinde 82 auf, welches sich in der gezeigten Ausführungsform über-die gesamte Höhe der Knochenplatte 1 von der Oberseite 81 zur Unterseite 83 erstreckt.

In das Schwenkloch 12 ist die als Knochenschraube ausgeführte Schwenkschraube 2 eingeführt, derart, dass der als Kugelkopf ausgeführte Schraubenkopf 20 der Schwenkschraube 2 im Wesentlichen formschlüssig in der Kugelkopfaufnahme 80 liegt. Der Schraubenkopf 20 der Schwenkschraube 2, die auch als Kugelkopfschraube bezeichnet werden kann, ist mit einer Gewindekontur 84 versehen, die mit im Wesentlichen horizontal umlaufenden Rillen 85, einem Rechts- sowie einem Linksgewinde 86, 87 gebildet ist. Es kann vorgesehen sein, dass das Rechts-und/oder Linksgewinde 86, 87 mehrgängig ausgeführt sind.

In das Schraub- oder Klemmloch 13 ist die ebenfalls als Knochenschraube ausgeführte Klemmschraube 3 eingedreht, die am Schraubenkopf 30 über ein konisches Außengewinde 88 verfügt, welches in das zylindrische Innengewinde 82 des Schraub- oder Klemmloches 13 eingedreht ist. Die Klemmschraube 3 ist aufgrund des Zusammenwirkens des zylindrischen Innengewindes 82 und des konischen Außengewindes 88 in seiner Relativlage zur Knochenplatte 1 im Wesentlichen festgelegt, insbesondere hinsichtlich einer räumlichen Winkelstellung zur Knochenplatte 1.

Einander gegenüberliegende Gewindeabschnitte der Gewindekontur 84 einerseits sowie des konischen Außengewindes 88 andererseits greifen bei dem in Fig. 1 dargestellten eingeschraubten Zustand formschlüssig ineinander. Die Schraubenköpfe 20, 30 der Schwenkschraube 2 und der Klemmschraube 3 sind im eingedrehten Zustand miteinander sowie mit der Knochenplatte 1 verklemmt, sodass sie mehrdimensional winkelstabil fixiert sind.

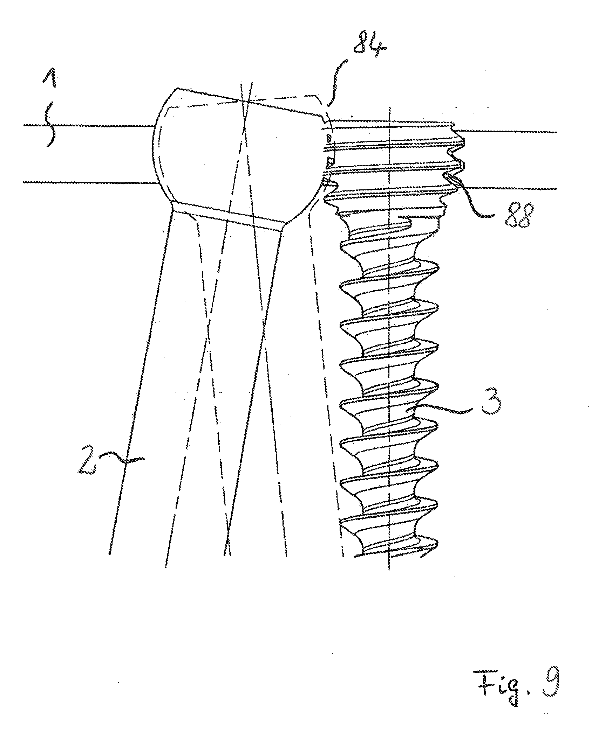

Fig. 9 zeigt schematisch unterschiedliche Winkel- oder Schwenkstellungen der Schwenkschraube 2, wobei in jeder der Schwenkstellungen (durchgehende und gestrichelte Linien) Gewindeabschnitte der Gewindekontur 84 sowie des konischen Außengewindes 88 formschlüssig ineinandergreifen.

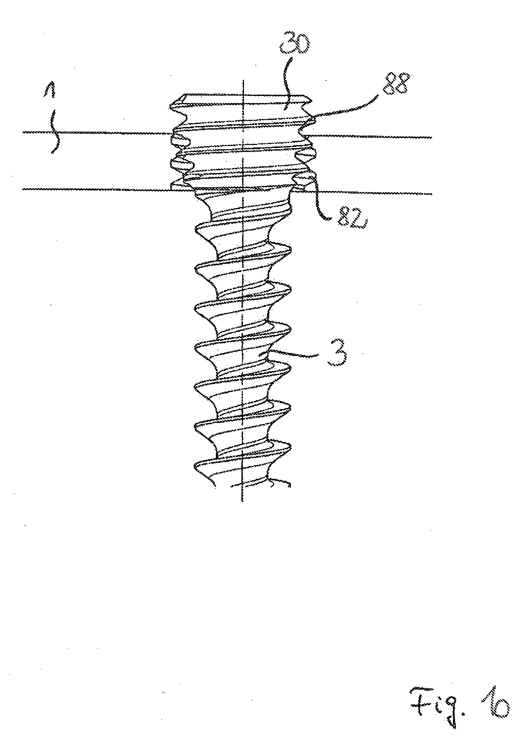

Fig. 10 zeigt eine schematische Darstellung betreffend das Zusammenwirken des konischen Außengewindes 88 sowie des zylindrischen Innengewindes 82 beim Eindrehen der Klemmschraube 3.

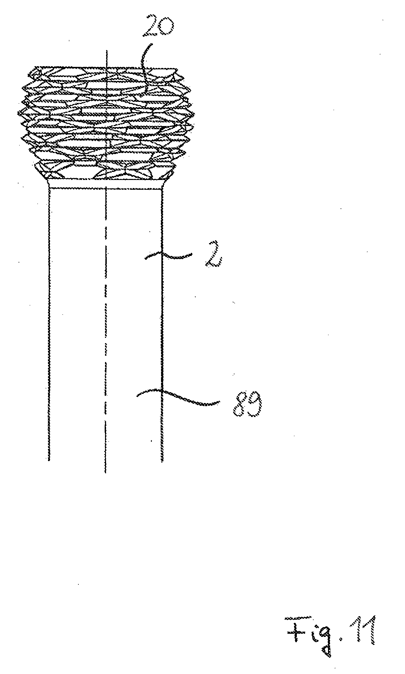

Fig. 11 zeigt eine schematische Darstellung für die Schwenkschraube 2, wobei wie in den Fig. 1 und 2 das Knochengewinde auf dem Schaft 89 der Schwenkschraube 2 nicht dargestellt ist. Dieses kann beispielsweise in gleicher Weise wie bei der Klemmschraube 3 ausgeführt sein (vgl. hierzu Fig. 8 bis 10). Schließlich zeigt

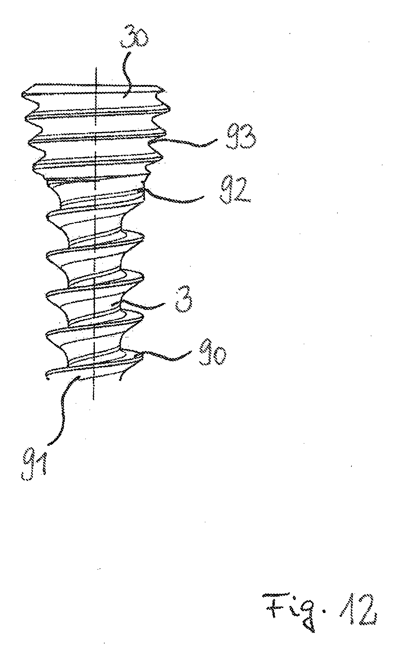

Fig. 12 eine schematische Darstellung der Klemmschraube 3. Benachbart zum Schraubenkopf 30 ist ein Knochengewinde 90 auf dem Schaft 91 der Klemmschraube 3 mit einem sich erweiternden Abschnitt 92 und sich an den Gewindegrund 93 des konischen Außengewindes 88 im Wesentlichen anschließenden Übergangsbereich gebildet.

Weitere mechanische Veränderungen der Oberflächen von Schwenkloch, Schwenkschraube, Klemmschraube und Klemmloch können einen Reibschluss und / oder Formschluss zwischen Schrauben und / oder zwischen Schraube und Knochenplatte erheblich beeinflussen Zu solchen mechanischen Veränderungen zählen unter anderem:
- Verwendung unterschiedlicher Implantatmaterialien wie Reintitan, Titanlegierungen und andere Implantatstahl-Metalllegierungen.
- Unterschiedliche Oberflächenhärtung verschiedener Komponenten (zum Beispiel besondere Härte der Fixschraube).
- Radiäre Gewinde oder radiäre Stege geringer Höhe auf der Oberfläche des Schwenkloches und / oder des Klemmloches. Selbstverständlich können ein oder mehrere Gewinde oder Stege in beliebig anderen Ausrichtungen ausgeführt werden.
- Längsgewinde oder Längsstege geringer Höhe auf der Oberfläche der Schwenkschraube und / oder der Fixschraube. Selbstverständlich können ein oder mehrere Gewinde oder Stege in beliebig anderen Ausrichtungen ausgeführt werden.
- Oberflächenrauhigkeit des Schwenkloches und / oder des Klemmloches und / oder des Schwenkschraubenkopfes und / oder des Fixschraubensenkkopfes (zum Beispieldurch Sandstrahlen).
- Weiche Oberfläche des Rundkopfes der Schwenkschraube (damit ein härteres Gewinde des Konus der Klemmschraube sich in den weicheren Kugelkopf einkerben kann).
- Ummantelung des Kugelkopfes der Schwenkschraube mit einem biokompatiblem nichtmetallischem Material (beispielsweise Polypropylen) oder einem Übergangsmetall (beispielsweise Tantal). Es ist klar, dass auch resorbierbare biokompatible Materialien für die Ummantelung des Kugelkopfes verwendet werden können (beispielsweise Laktid). Durch die Ummantelung des Kugelkopfes der Schwenkschraube kann sich ein Gewinde am Fixschraubenkopf formschlüssig in den weicheren Mantel auf dem Schwenkschraubenkopf eingraben. Das biokompatible Material sollte eine raue und verschleißarme Oberfläche aufweisen.
- Veränderung der Gewindeformen wie zum Beispiel PG-Gewinde, NPT-Gewinde, Trapezgewinde, Kugelgewinde.
- Veränderung der Fixschraubengeometrie wie zum Beispiel Ausgestaltung als Linsenkopf oder Birnenkopf oder Veränderung der Schraubenkopfgeometrie im Sinne einer zum Schraubenkopf konkaven oder konkavähnlichen Freiform der Kopfgeometrie, um einen Formschluss zum Kopf der Schwenkschraube zu erreichen.

Um während einer Operation eine möglichst einfache Handhabung des neuen Platten-Schrauben-Systems zu erreichen, wird außerdem eine vorteilhafte Markierung der Platten und Schrauben vorgeschlagen. Hierdurch soll eine möglichst einfache Anlagerung der Knochenplatte und Besetzung von Schraubenlöchern mit Knochenschrauben erreicht werden. Die Kennzeichnung oder Markierung kann beispielsweise durch Gravur und / oder Drucktechnik und / oder galvanisch erfolgen.

Das Knochenplattensystem kann außerdem in vorteilhaften Ausgestaltungen ein oder mehrere der folgenden Merkmale aufweisen:
- Anatomisch geformte Platten in Gelenkbereichen von Röhrenknochen, zum Beispiel von langen Röhrenknochen der Extremitäten
- Materialausgleich zwischen Plattenlöchern oder Plattenlochgruppen, um intraoperativ eine Verbiegungen im Zwischenlochbereich günstig zu ermöglichen
- Kleine Löcher in Platten, um (i) eine orientierende Fixierung der Knochenplatte an den Knochen, zum Beispiel mit Hilfe von Drähten, zu ermöglichen und (ii) Fäden oder Anker an die Knochenplatte fixieren zu können.

Häufig können Knochenplatten schonend mit Hilfe von Zielbügeln minimalinvasiv eingebracht werden. Um das Platten-Schrauben-System der vorgeschlagenen Erfindung möglichst sicher einzubringen, sind außerdem besonders ausgebildete Zielbügel wünschenswert. Nachfolgende Merkmale kennzeichnen unter anderem einen für das Knochenplattensystem vorteilhaft ausgebildeten Zielbügel:
- Zielbügel mit Haltevorrichtung für die Platte.
- Haltevorrichtung für die Knochenplatte kann beispielsweise mit Hilfe von Gewinden an Hülsen, welche in einem Klemmloch oder mehreren Klemmlöchern greifen, an der Knochenplatte befestigt werden
- Zielbügel enthält ein oder mehrere kleine Zusatzlöcher für Drähte zur vorübergehenden Plattenfixation
- Zielbügel ist durch unterschiedliche Markierungen für Schwenkschrauben und Fixschrauben gekennzeichnet. Markierungen können beispielsweise durch Gravur und / oder Drucktechnik erzeugt werden.
- An den Zielbügel können eine oder mehrere aufsetzbare Backen zum Einbringen von Schwenklöchern fixiert werden. Eine aufsetzbare Backe ist mit einer Vorrichtung versehen, welche als variabel einstellbare Zielvorrichtung für Schwenkschrauben verwendet werden kann. Die Zielvorrichtung führt beispielsweise eine Buchse (zur Aufnahme von Bohrhülsen oder Hülsen zur Schraubenführung) senkrecht auf einer gekrümmten Fläche eines Kugelsegmentes, dessen Kugelmittelpunkt mit dem Mittelpunkt des Schwenkschraubenloches übereinstimmt oder annähernd übereinstimmt.

Die Knochenplatte kann vorteilhaft bei Radiusbrüchen, Humerusbrüchen, Femurbrüchen oder Tibiabrüchen eingesetzt werden. Um in Gelenknähe häufig verwendete Bohrrichtungen und Schraubenlängen vorgeben zu können, ist ein Plattenaufsatz wünschenswert, der günstige Bohrrichtungen für Schwenklöcher und Klemmlöcher berücksichtigt. Merkmale eines hierfür besonders geeigneten Plattenaufsatzes sind:
- Der Plattenaufsatz hat eine vielfache Dicke der Knochenplatte (zum Beispiel 3fach) und enthält Bohrungen in Richtungen, welche typischerweise oder günstig in der jeweiligen Gelenkregionen verwendet werden können
- Der Plattenaufsatz enthält Markierungen für Schwenklöcher und Klemmlöcher
- Der Plattenaufsatz kann mit Kennzeichnungen für häufige Schraubenlängen versehen sein.
   Die Kennzeichnung kann beispielsweise durch Gravur und / oder Drucktechnik angebracht werden.
- Der Plattenaufsatz enthält ein oder mehrere zusätzliche Löcher für Drähte zur temporären Befestigung der Knochenplatte an den Knochen.
- Ein Klemm- oder Schraubmechanismus erlaubt eine schnelle Kopplung des Plattenaufsatzes an die Platte.

## Patentansprüche

1. Knochenplattensystem zur Osteosynthese, mit:
- einer Knochenplatte (1),
- einer Schwenkschraube (2),
- einer Klemmschraube (3),
- einem Schwenkloch (12), welches in der Knochenplatte (1) als Durchgangsloch zur polyaxialen Aufnahme der Schwenkschraube (3) gebildet ist, und
- einem dem Schwenkloch (12) zugeordneten Klemmloch (13), welches in der Knochenplatte (1) als weiteres Durchgangsloch zur Aufnahme der Klemmschraube (3) gebildet ist,
wobei im eingeschraubten Zustand die Schwenkschraube (2) und die Klemmschraube (3) mehrdimensional winkelstabil fixiert sind, indem Schraubenköpfe (20, 30) der Schwenkschraube (2) und der Klemmschraube (3) mittels gegenseitigem Abstützen oder Klemmen untereinander sowie mit der Knochenplatte (1) gegen eine Relativbewegung gesichert sind, und wobei die Schwenkschraube (2) und die Klemmschraube (3) jeweils als eine Knochenschraube ausgeführt sind.

2. Knochenplattensystem nach Anspruch 1, **dadurch gekennzeichnet, dass** das Schwenkloch (12) mit einer sich zur Oberseite der Knochenplatte (1) hin öffnenden Kugelkopfaufnahme gebildet und die Schwenkschraube (2) einen zugeordneten Kugelkopf aufweist, der im eingeschraubten Zustand der Schwenkschraube (2) wenigstens teilweise in der Kugelkopfaufnahme des Schwenkloches (12) angeordnet ist.

3. Knochenplattensystem nach Anspruch 2, **dadurch gekennzeichnet, dass** die Kugelkopfaufnahme wenigstens abschnittsweise mit einer im Wesentlichen glatten Oberfläche und / oder wenigstens abschnittsweise mit einer Oberflächenkontur gebildet ist.

4. Knochenplattensystem nach mindestens einem der vorangehenden Ansprüche, **dadurch gekennzeichnet, dass** das Klemm loch (13) ein Innengewinde aufweist.

5. Knochenplattensystem nach mindestens einem der vorangehenden Ansprüche, **dadurch gekennzeichnet, dass** der Schraubenkopf (30) der Klemmschraube (3) ein Außengewinde aufweist.

6. Knochenplattensystem nach den Ansprüchen 2 und 5, **dadurch gekennzeichnet, dass**
- der Schraubenkopf (20) der Schwenkschraube (2) eine Gewindekontur (84) aufweist, die mit im Wesentlichen horizontal umlaufenden Rillen, einem Rechts- sowie einem Linksgewinde gebildet ist,
- das Außengewinde des Schraubenkopfes (30) der Klemmschraube (3) im eingeschraubten Zustand der Klemmschraube (3) wenigstens teilweise in das Innengewinde des Klemmloches (13) eingedreht ist, und
- im eingeschraubten Zustand Oberflächenabschnitte der Gewindekontur am Schraubkopf (20) der Schwenkschraube (2) und des Außengewindes am Schraubenkopf (30) der Klemmschraube (3) formschlüssig ineinander greifen.

7. Knochenplattensystem nach Anspruch 5 oder 6, **dadurch gekennzeichnet, dass** das Außengewinde am Schraubenkopf (30) der Klemmschraube (3) ein konisches Außengewinde ist, welches sich am Schraubenkopf (30) in Einschraubrichtung verjüngt.

8. Knochenplattensystem nach Anspruch 7, **dadurch gekennzeichnet, dass** das konische Außengewinde mit einer Gewindesteigung gebildet ist, die geringer als die Gewindesteigung am Schaft der Klemmschraube (3) ist.

9. Knochenplattensystem nach mindestens einem der vorangehenden Ansprüche, **dadurch gekennzeichnet, dass** das zwischen dem Schwenkloch (12) und dem dem Schwenkloch (12) zugeordneten Klemmloch (13) ein Lochübergang mit einem Durchbruch gebildet ist.

10. Knochenplattensystem nach mindestens einem der vorangehenden Ansprüche, soweit rückbezogen auf die Ansprüche 4 und 5, **dadurch gekennzeichnet, dass** die Knochenplatte (1) mit einem an das Klemmloch (13) angrenzenden Dehnungsbereich gebildet ist, derart, dass die Knochenplatte (1) beim Einschrauben der Klemmschraube (3) in das Innengewinde des Klemmloches (13) dem Einschraubdruck aufgrund der Wechselwirkung zwischen dem Außengewinde und dem Innengewinde zumindest teilweise nachgebend und frei von plastischer Verformung verformbar ist.

11. Knochenplattensystem nach mindestens einem der vorangehenden Ansprüche, **dadurch gekennzeichnet, dass** bei der Klemmschraube (3) der Schraubenschaft eine Länge aufweist, die gleich der oder kürzer als die Länge des Schraubenschaftes der Schwenkschraube (2) ist.

12. Knochenplattensystem nach mindestens einem der vorangehenden Ansprüche, soweit auf Anspruch 5 rückbezogen, **dadurch gekennzeichnet, dass** bei der Klemmschraube (3) ein Knochengewinde auf dem Schraubenschaft benachbart zum Schraubenkopf (30) mit einem sich erweiternden Abschnitt und sich an den Gewindegrund des Außengewindes im Wesentlichen anschließenden Übergangsbereich gebildet ist.

13. Knochenplattensystem nach mindestens einem der vorangehenden Ansprüche, **dadurch gekennzeichnet, dass**
- in der Knochenplatte (1) mindestens ein weiteres dem Schwenkloch (12) oder dem Klemmloch (13) entsprechend ausgeführtes und zum Schwenkloch (12) und zum Klemmloch (13) korrespondierendes Durchgangsloch gebildet ist,
- in das weitere Durchgangsloch eine weitere Knochenschraube eingeführt ist, die der Schwenkschraube (2) oder der Klemmschraube (3) entsprechend ausgeführt ist, und
- der Schraubenkopf der weiteren Knochenschraube im eingeschraubten Zustand mit den Schraubenköpfen (20, 30) sowie der Knochenplatte (1) gegen eine Relativbewegung gesichert ist.

## Claims

1. A bone plate system for osteosynthesis with
- a bone plate (1),
- a swivel screw (2),
- a locking screw (3),
- a swivel hole (12) which is formed in the bone plate (1) as a through hole for polyaxially receiving the swivel screw (3), and
- a locking hole (13) which is assigned to the swivel hole (12) and is formed in the bone plate (1) as a further through hole for receiving the locking screw (3)
wherein in a screwed-in state the swivel screw (2) and the locking screw (3) are multi-dimensionally fixed in an angular stable manner in that the screw heads (20, 30) of the swivel screw (2) and the locking screw (3) are secured against a relative movement by way of being supported or clamped to each other and to the bone plate (1), and wherein the swivel screw (2) and the locking screw (3) are each designed as a bone screw.

2. The bone plate system according to claim 1, **characterised in that** the swivel hole (12) is formed with a ball head receptacle opening towards the upper side of the bone plate (1) and the swivel screw (2) has a corresponding ball head which in the screwed-in state of the swivel screw (2) is at least partially arranged in the ball head receptacle of the swivel hole (12).

3. The bone plate system according to claim 2, **characterised in that** the ball head receptacle is formed at least in sections with an essentially smooth surface and/or at least in sections with a surface contour.

4. The bone plate system according to at least one of the preceding claims, **characterised in that** the locking hole (13) has an internal thread.

5. The bone plate system according to at least one of the preceding claims, **characterised in that** the screw head (30) of the locking screw (3) has an external thread.

6. The bone plate system according to claims 2 and 5, **characterised in that**
- the screw head (20) of the swivel screw (2) has a thread contour (84) that is formed with essentially horizontal circumferential grooves as well as a right-handed and a left-handed thread,
- when the locking screw (3) is screwed in, the external thread of the screw head (30) of the locking head (3) is at least partially screwed into the internal thread of the locking hole (13), and
- in the screwed-in state, surface sections of the thread contour on the screw head (20) of the swivel screw (2) and the external thread of the screw head (30) of the locking screw (3) positive interlock with each other.

7. The bone plate system according to claim 5 or 6, **characterised in that** the external thread on the screw head (30) of the locking screw (3) is a conical external thread which on the screw head (30) tapers in the direction of screwing in.

8. The bone plate system according to claim 7, **characterised in that** the conical external thread is designed with a thread pitch that is smaller than the thread pitch on the shaft of the locking screw (3).

9. The bone plate system according to at least one of the preceding claims, **characterised in that** between the swivel hole (12) and the locking hole (13) associated with the swivel hole (12) there is a hole transition with an aperture.

10. The bone plate system according to at least one of the preceding claims, insofar as it refers back to claims 4 and 5, **characterised in that** the bone plate (1) is formed with an expansion area adjoining the locking hole (13) in such a way that on screwing the locking screw (3) into the internal thread of the locking hole (13) the bone plate (1) at least in parts yields to the screwing in pressure due to the interaction between the external thread and the internal thread and is deformable and free of plastic deformation.

11. The bone plate system according to at least one of the preceding claims, **characterised in that** the locking screw (3) of the screw shaft has a length which is equal to or shorter than the length of the screw shaft of the swivel screw (2).

12. The bone plate system according to at least one of the preceding claims, insofar as it refers back to claim 5, **characterised in that** on the locking screw (3) a bone thread is formed on the screw shaft adjacent to the screw head (30) with a widening section and transition area essentially adjoining the thread base of the external thread.

13. The bone plate system according to at least one of the preceding claims, **characterised in that**
- in the bone plate (1) at least one further through hole is formed which is designed in correlation to the swivel hole (12) or the locking hole (13) and corresponds with the swivel hole (12) or the locking hole (13),
- a further bone screw is introduced into the further through hole which is designed in accordance with the swivel screw (2) of the locking screw (3) and
- in the screwed-in state the screw head of the further bone screw is secured with the screw heads (20, 30) as well as the bone plate (1) against a relative movement.

## Revendications

1. Système de plaque osseuse pour ostéosynthèse, comportait
- une plaque osseuse (1),
- une vis de pivotement (2),
- une vis de serrage (3),
- un trou de pivotement (12) qui est pratiqué dans la plaque osseuse (1) sous forme d'un trou traversant destiné à recevoir polyaxialement la vis de pivotement (3) et
- un trou de serrage (13) associé au trou de pivotement (12) et qui est pratiquée dans la plaque osseuse (1) sous forme d'un autre trou traversant destiné à recevoir la vis de serrage (3),
dans lequel, en état vissé, la vis de pivotement (2) et la vis de serrage (3) sont fixées avec une stabilité angulaire multidimensionnelle en bloquant les têtes de vis (20, 30) de la vis de pivotement (2) et de la vis de serrage (3) par appui ou serrage réciproque ainsi qu'avec la plaque osseuse (1) pour éviter tout mouvement relatif et dans lequel la vis pivotement (2) et la vis de serrage (3) sont réalisés respectivement sous forme d'une vis à os.

2. Système de plaque osseuse selon la revendication 1, **caractérisé en ce que** le trou de pivotement (12) est réalisé avec un support de tête sphérique ouvrant sur la face supérieure de la plaque osseuse (1) et que la vis de pivotement (2) présente une tête sphérique associée qui, en état vissé de la vis de pivotement (2), est disposée du moins partiellement dans le support de tête sphérique du trou de pivotement (12).

3. Système de plaque osseuse selon la revendication 2, **caractérisé en ce que** le support de tête sphérique est réalisé du moins par endroits avec une surface sensiblement lisse et/ou du moins par endroits avec un contour surfacique.

4. Système de plaque osseuse selon au moins une des revendications précédentes, **caractérisé en ce que** le trou de serrage (13) présente un filetage intérieur.

5. Système de plaque osseuse selon au moins une des revendications précédentes, **caractérisé en ce que** la tête de vis (30) de la vis de serrage (3) présente un filetage extérieur.

6. Système de plaque osseuse selon les revendications 2 et 5, **caractérisé en ce que**
- la tête de vis (20) de la vis de pivotement (2) présente un contour fileté (84) qui est constitué de rainures sensiblement périphériques horizontalement, d'un filet droit et d'un filet gauche,
- le filetage extérieur de la tête de vis (30) de la vis de serrage (3), en état vissé de la vis de serrage (3), est vissé du moins partiellement dans le filetage intérieur du trou de serrage (13) et,
- en état vissé, des sections surfaciques du contour fileté s'engrènent les unes dans les autres par correspondance géométrique au niveau de la tête de vis (20) de la vis de pivotement (2) et du filetage extérieur au niveau de la tête de vis (30) de la vis de serrage (3).

7. Système de plaque osseuse selon la revendication 5 ou 6, **caractérisé en ce que** le filetage extérieur de la tête de vis (30) de la vis de serrage (3) est un filetage extérieur conique qui se rétrécit au niveau de la tête de vis (30) dans le sens de vissage.

8. Système de plaque osseuse selon la revendication 7, **caractérisé en ce que** le filetage extérieur conique est formé avec un pas de filetage qui est inférieur au pas de filetage de la tige de la vis de serrage (3).

9. Système de plaque osseuse selon au moins une des revendications précédentes, **caractérisé en ce que**, entre le trou de pivotement (12) et le trou de serrage (13) associé au trou de pivotement (12), une transition de trous est formée par le biais d'une percée.

10. Système de plaque osseuse selon au moins une des revendications précédentes dans la mesure où elles font référence aux revendications 4 et 5, **caractérisé en ce que** la plaque osseuse (1) est réalisé avec une zone d'extension adjacente au trou de serrage (13) de manière à ce que la plaque osseuse (1), lors du vissage de la vis de serrage (3) dans le filetage intérieur du trou de serrage (13), soit du moins partiellement déformable en cédant à la pression de serrage en raison de l'interaction entre le filetage extérieur et le filetage intérieur et sans déformation plastique.

11. Système de plaque osseuse selon au moins une des revendications précédentes, **caractérisé en ce que**, sur la vis de serrage (3), la tige de vices à une longueur qui est égale ou inférieure à la longueur de la tige de vis de la vis de pivotement (2).

12. Système de plaque osseuse selon au moins une des revendications précédentes, dans la mesure où elle fait référence à la revendication 5, **caractérisé en ce que**, sur la vis de serrage (3), un filetage douces et réalisé sur la tige de vis à proximité de la tête de vis (30) avec une section s'évasant et se raccordant substantiellement à la base du filet du filetage extérieur.

13. Système de plaque osseuse selon au moins une des revendications précédentes, **caractérisé en ce que**
- dans la plaque osseuse (1), est pratiqué au moins un autre trou traversant réalisé en fonction du trou de pivotement correspondant (12) ou du trou de serrage (13) et correspondant au trou de pivotement (12) et au trou de serrage (13),
- dans l'autre trou traversant est introduite une autre vis à os qui est réalisé en fonction de la vis de pivotement (2) ou de la vis de serrage (3) et que
- la tête de vices de l'autre vis à os, en état vissé, est bloquée par les têtes de vis (20, 30) et la plaque osseuse (1) pour éviter tout mouvement relatif.
